# EUROPÄISCHE PATENTANMELDUNG

(11) **EP 1 138 309 A2**
(43) Veröffentlichungstag der Anmeldung: **04.10.2001**
(21) Anmeldenummer: 01107564.5
(22) Anmeldetag: 27.03.2001
(51) Int. Cl.: A61K 6/087

(54) **Dentalwerkstoff auf Basis von kationisch polymerisierbaren Monomeren**

(30) Priorität: 31.03.2000 DE 10016323
(71) Anmelder: Degussa Dental GmbH & Co. KG, 63457 Hanau (DE)
(72) Erfinder: Albert, Philipp, Dr., 79539 Lörrach (DE); Loehden, Gerd, Dr., 63457 Hanau (DE); Gall, Corina, 36381 Schlüchtern (DE); Rentsch, Harald, Dr., 63457 Hanau (DE)

(57) **Zusammenfassung**

Die vorliegende Erfindung betrifft Dentalwerkstoffe auf Basis von kationisch polymerisierbaren, Monomeren als Bindemittel, eines Polymerisationsinitiators und bezogen auf den Dentalwerkstoff 1-95 Gew.-% mindestens eines anorganischen Füllstoffs, wobei das Bindemittel Monomere der Formel (I) enthält 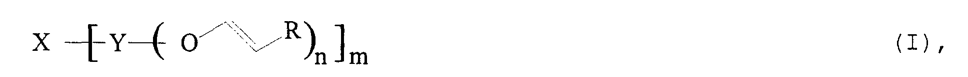 worin R Wasserstoff, eine Methyl- oder Ethylgruppe, X und Y jeweils unabhängig einen unsubstituierten oder substituierten aliphatischen, cycloaliphatischen oder aromatischen Rest mit 1 bis 100 Kohlenstoffatomen, wobei eine oder mehrere CH₂-Gruppen durch O, C=O, -CO₂-, -SiR¹₂- und/ oder -SiR¹₂O-, worin R¹ unabhängig ein Alkyl- oder Alkoxy- oder Arylrest mit 1 bis 10 C-Atomen bedeutet, ersetzt sein können, n eine ganze Zahlen von 1 bis 3 und m eine ganze Zahl von 2 bis 5 darstellen.

Die neuen Dentalmassen zeichnen sich durch eine besonders geringe durch die Härtung bedingte Volumenabnahme und besonders gute mechanische Eigenschaften sowie kurze Polymerisationszeiten aus.

## Beschreibung

Die vorliegende Erfindung betrifft Dentalwerkstoffe auf Basis von kationisch polymerisierbaren Monomeren sowie Verfahren zu deren Herstellung.

Bislang vorwiegend verwendete polymerisierbare Dentalwerkstoffe enthalten meist bi- oder polyfunktionelle Acrylate und Methacrylate, welche radikalisch gehärtet werden.

Solche Werkstoffe sind beispielsweise in EP-A-0 091 990 beschrieben. Ein großer Nachteil der bekannten polymerisierbaren Dentalmassen ist der Polymerisationsschrumpf, der beispielsweise bei der Anwendung als Füllmaterial durch die Bildung von Randspalten Sekundärkaries verursachen kann. Zur Verbesserung dieses Schrumpfes wurde in EP-A-0 754 675 vorgeschlagen Monomere zu verwenden, die flüssigkristalline Eigenschaften aufweisen. Diese Maßnahme führt zwar zu einer spürbaren Verbesserung der Volumenabnahme, diese ist aber für viele Bereiche nicht ausreichend.

Des weiteren sind polymerisierbare Massen auf Basis von Epoxiden bekannt, die als Dentalwerkstoff eingesetzt werden können. Solche Dentalwerkstoffe sind beispielsweise in WO 96/13 538, WO 95/30 402 und WO 98/22 521 beschrieben.

Nachteilig an diesen Epoxiden ist zunächst die geringe Geschwindigkeit der Härtung. Diese führt u.a. zu einem längeren Einbringen des Füllguts in die vom Zahnarzt gelegte Kavität. Ein weiterer Nachteil von Dentalwerkstoffen, die auf Epoxiden basieren, ist deren geringe Lagerbeständigkeit. Epoxide lassen sich sowohl kationisch mit Säuren als auch anionisch durch Basen polymerisieren. Daher ist es nicht möglich durch Zugabe von Inhibitoren die Lagerfähigkeit wesentlich zu erhöhen. Während Methacrylate durch Zugabe von Radikalfängern gelagert werden können, führt sowohl die Zugabe von Basen als auch von Säure zu einer Polymerisation der Epoxide.

Nachteilig ist weiterhin die relativ hohe Viskosität von Epoxiden, die auf deren Polarität zurückzuführen ist. Infolge dieser hohen Viskosität lassen sich nur relativ geringe Anteile an Füllstoffen einarbeiten oder man muß Monomere zur Verdünnung einsetzen. Dies führt unweigerlich zu relativ ungünstigen mechanischen Eigenschaften der ausgehärteten Dentalwerkstoffe.

Darüber hinaus sind viele Epoxide in hohem Maße zytotoxisch. So zeigten in-vitro Tests, daß die meisten dieser Monomere mutagene Eigenschaften haben.

In Anbetracht des hierin angegebenen und diskutierten Standes der Technik war es mithin Aufgabe der Erfindung, einen verbesserten Dentalwerkstoff anzugeben, der insbesondere einen verringerten Schrumpf und eine hohe Geschwindigkeit der Härtung zeigt.

Weiterhin ist Aufgabe der Erfindung, die Angabe von Bindemitteln, die eine hohe Lagerbeständigkeit und eine geringe Toxizität zeigen. Die Bestandteile des Bindemittels, insbesondere die Monomere, sollten des weiteren leicht erhältlich und/oder einfach herstellbar sein.

Des weiteren sollten alle an einen modernen Dentalwerkstoff gestellten Anforderungen hinsichtlich Transparenz, Polierbarkeit, Druckfestigkeit, Wasseraufnahme, Abriebfestigkeit, Biegefestigkeit, Röntgenopazität usw. in besonders hohem Maße erfüllt werden.

Die Lösung dieser sowie weiterer im einzelnen nicht näher genannten, jedoch aus den einleitenden Erörterungen des Standes der Technik ableitbaren oder sich ohne weiteres erschließenden Aufgaben gelingt durch einen Dentalwerkstoff der eingangs erwähnten Gattung, welcher die Merkmale des kennzeichnenden Teils des Anspruches 1 aufweist. Zweckmäßige Weiterbildungen werden in den von Anspruch 1 abhängigen Ansprüchen unter Schutz gestellt. Hinsichtlich des Verfahrens zur Herstellung bietet Anspruch 14 einen Vorschlag für die Lösung des der Aufgabe zugrundeliegenden Problems an.

Dadurch, daß das Bindemittel Monomere der Formel (I) enthält, 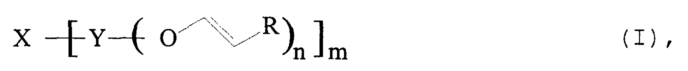 worin R Wasserstoff, eine Methyl- oder Ethylgruppe, X und Y jeweils unabhängig einen unsubstituierten oder substituierten aliphatischen, cycloaliphatischen oder aromatischen Rest mit 1 bis 100 Kohlenstoffatomen, wobei eine oder mehrere CH₂-Gruppen durch O, C=O, -CO₂-, -SiR12-und/ oder -SiR¹₂O-, worin R¹ unabhängig ein Alkyl- oder Alkoxy- oder Arylrest mit 1 bis 10 C-Atomen bedeutet, ersetzt sein können, n eine ganze Zahlen von 1 bis 3 und m eine ganze Zahl von 2 bis 5 darstellen, gelingt es, einen Dentalwerkstoff auf Basis von kationisch polymerisierbaren Monomeren als Bindemittel, eines Polymerisationsinitiators und bezogen auf den Dentalwerkstoff 1 bis 95 Gew.-%, mindestens eines anorganischen Füllstoffes zu schaffen, der einen geringen Schrumpf bei gleichzeitig hoher Lagerfähigkeit zeigt.

Durch die erfindungsgemäßen Maßnahmen werden u.a. folgende Vorteile erzielt:
⇒ Die gehärteten Dentalmassen weisen eine hohe Transparenz, eine ausgezeichnete Polierbarkeit, eine sehr gute Druckfestigkeit, eine äußerst geringe Wasseraufnahme, eine hohe Abriebfestigkeit, eine sehr hohe Biegefestigkeit und eine hohe Röntgenopazität auf.
⇒ Darüber hinaus zeigen die Dentalwerkstoffe eine hohe Lagerfähigkeit, eine hohe Geschwindigkeit der Härtung, eine gute Verarbeitbarkeit und eine geringe Toxizität.
⇒ Ein weiterer Vorteil der erfindungsgemäßen Dentalwerkstoffe besteht darin, daß die Polymerisation nur angestoßen werden muß, um anschließend selbständig weiterzulaufen.
⇒ Des weiteren ist der Schrumpf der erfindungsgemäßen Dentalwerkstoffe sehr gering.
⇒ Weiterhin sind Monomere der Formel (I) relativ einfach und kostengünstig erhältlich.

Dentalwerkstoff bezeichnet im Rahmen der Erfindung Materialien für die Zahnfüllung, Inlays oder Onlays, Zahnzemente, Glasionomerzemente, Kompomere, Komposite, Verblendmaterialien für Kronen und Brücken, Materialien für künstliche Zähne, Dentinbondings, Unterfüllmaterialien, Wurzelfüllmaterialien oder sonstige Materialien für die prothetische, konservierende und präventive Zahnheilkunde. Insbesondere fallen unter den Begriff Dentalwerkstoff auch Komposits für zahnmedizinische und zahntechnische Einsatzzwecke, Versieglermaterialien, selbsthärtende Komposits, Stumpfaufbaumaterialien, Verblendkunststoffe, hoch- und normalgefüllte Dualzemente sowie normalgefüllte fluoridhaltige Zahnlacke.

Üblicherweise umfassen diese Dentalwerkstoffe Bindemittel, Polymerisationsinitiatoren sowie anorganische Füllstoffe.

Das Bindemittel erfindungsgemäßer Dentalwerkstoffe enthält Monomere der Formel (1) 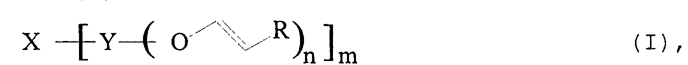 worin R Wasserstoff, eine Methyl- oder Ethylgruppe, X und Y jeweils unabhängig einen unsubstituierten oder substituierten aliphatischen, cycloaliphatischen oder aromatischen Rest mit 1 bis 100 Kohlenstoffatomen, wobei eine oder mehrere CH₂-Gruppen durch O, C=O, -CO₂-, -SiR12-und/ oder -SiR¹₂O-, worin R¹ unabhängig ein Alkyl- oder Alkoxy- oder Arylrest mit 1 bis 10 C-Atomen bedeutet, ersetzt sein können, n eine ganze Zahlen von 1 bis 3, vorzugsweise 1 oder 2, und m eine ganze Zahl von 2 bis 5, vorzugsweise 3 oder 4, darstellen.

Eine bevorzugte Gruppe von Monomeren der Formel (I) ist voluminös jedoch gleichzeitig starr. Voluminöse Monomere zeigen im allgemeinen einen besonders geringen Polymerisationsschrumpf. Hochmolekulare Stoffe erhöhen jedoch die Viskosität des Bindemittels, so daß hierdurch der Füllstoffgehalt des Dentalwerkstoffs beschränkt wird.

Dementsprechend weisen Monomere der Formel (I) aromatische Gruppen auf, die beispielsweise von Benzol, Biphenyl, Diphenylether, Bisphenol A (2,2-Bis(4-hydroxyphenyl)propan) und/oder Diphenylcarbonat abgeleitet sind. Diese Gruppen können auch von kondensierten aromatischen Ringsystemen, wie beispielsweise Naphthalin, Acenaphthylen, Acenaphthen, Fluoren, Phenanthren, Anthracen, Fluoranthen, Pyren, Benz[a]anthracen, Chrysen, abgeleitet sein und/oder Heteroatome, insbesondere Schwefel und/oder Sauerstoff, aufweisen. Beispiele hierfür sind Benzofurane, Benzothiophene, Dibenzofurane und Dibenzothiophene.

Diese Gruppen können insbesondere in Form von aromatischen Polyester-, aromatischen Polyethergruppen und/oder Polycarbonatgruppen vorliegen, wobei die Anzahl der Kohlenstoffatome die oben genannten Werte nicht übersteigen sollte.

Darüber hinaus sind auch cyclische, insbesondere polycyclische Alkylgruppen, die unter anderem von Norbornan, Adamantan, Norcaran, Pinan, Tricylen abgeleitet werden können, besonders geeignet.

Eine weitere Gruppe von Monomeren, die in dem Bindemittel vorhanden sein können, sind flexibel. Zu Gruppen die diese Monomere aufweisen können gehören u.a. Siloxan-, Alkyl-oder Polyetherreste.

In einer ganz besonders bevorzugten Ausführungsform werden beide Gruppen als Mischung verwendet. Durch diese Maßnahme können in einer nicht vorhersehbaren Weise die mechanischen Eigenschaften verbessert werden, wobei insbesondere zähharte Dentalwerkstoffe erhalten werden. Je nach Anwendungszweck kann das Mischungsverhältnis in weiten Bereichen schwanken. Im allgemeinen werden jedoch Mischungen eingesetzt, in denen das Verhältnis flexibles Monomer zu starrem Monomer im Bereich von 10:1 bis 1:100, insbesondere von 1:1 bis 1:10 liegt.

Eine ähnliche Wirkung läßt sich auch dadurch erhalten, daß Monomere sowohl flexible als auch starre Gruppen im Molekül aufweisen. So kann beispielsweise der Rest X starr sein, während Y flexibel ist.

Monomere der Formel (I) sind mindestens bifunktionell, wobei sich die Funktionalität aus dem Produkt von n und m ergibt. Vorzugsweise ist dieses Produkt ≥ 3, besonders bevorzugt im Bereich von 4 bis 6. Je höher die Funktionalität des Monomeren desto höher die Wahrscheinlichkeit des Einbaus in die Matrix.

Weiterhin ist eine hohe Funktionalität notwendig, um eine hohe Vernetzungsdichte der Kunststoffmatrix zu erzielen. Dies führt zu Werkstoffen mit hoher Festigkeit und Haltbarkeit. Mit zunehmender Vernetzungsdichte neigen die Kunststoffe allerdings zur Sprödigkeit, die u.U. mit Nachteilen behaftet sein kann.

Beispiele bevorzugter Gruppen, die der Rest X einer Verbindung der Formel I aufweist sind u.a. 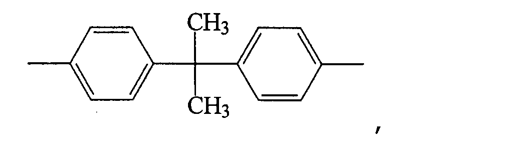 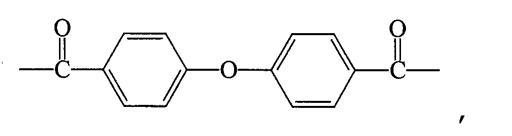 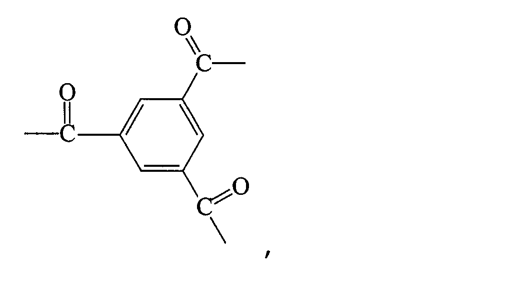 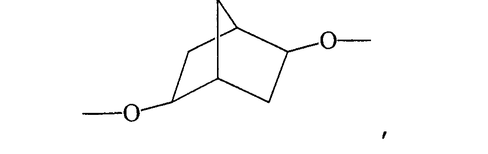 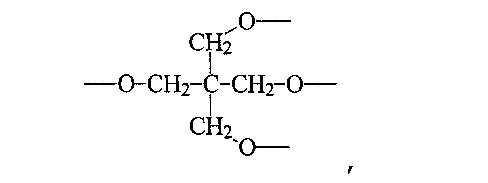 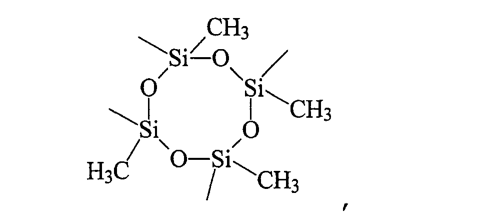 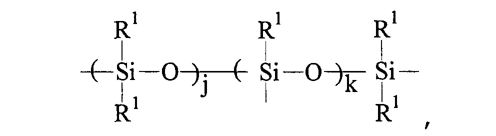 worin R¹ unabhängig ein Alkyl- oder Alkoxyrest mit 1 bis 6 C-Atomen, j und k unabhängig ganze Zahlen im Bereich von 1 bis 10 darstellen.

Spezielle Beispiele und bevorzugte Ausführungsformen der Reste Y sind 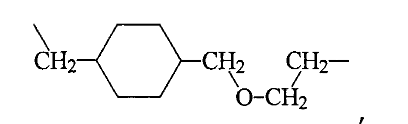 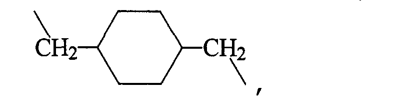 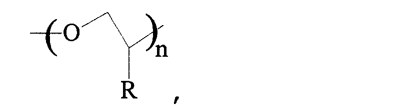 worin R Wasserstoff oder Methyl und n eine ganze Zahl im Bereich von 1 bis 10 darstellt.

Die Reste X und Y weiterhin können Substituenten aufweisen. Zu diesen Substituenten gehören u.a. Alkylgruppen, wie beispielsweise Methyl, Ethyl, Propyl, Butyl, 2-Propenyl, 2-Butenyl, Arylreste, wie beispielsweise Phenyl, Cumyl, Xylyl, Naphtyl, Alkoxyreste, wie Methoxy, Ethoxy, Propoxy.

Diese Reste können des weiteren halogenhaltige Gruppen, Estergruppen, Hydroxygruppen, Ethergruppen, Aminogruppen, Amidgruppen, Urethangruppen und Kohlensäuregruppen umfassen. Allerdings ist der Anteil basischer Gruppen, hierzu gehören unter anderem Aminogruppen, Amidgruppen und Urethangruppen, dadurch beschränkt, daß diese Gruppen eine nachteilige Wirkung auf die kationische Polymerisation haben können.

Spezielle Beispiele für Monomere der Formel (I) sind unter anderem 1,3,5-Benzoltricarbonsäure-tris-4-(ethenyloxy)butanolester; Butandisäure-bis-(4-ethenyloxy)butanolester; Bis-[(4-((ethenyloxy)methyl)-cyclohexyl)methyl]pentandioat; 2,2-Bis[4,1-phenyloxy-4-((ethenyloxy)methyl)cyclohexyl)methyl]propan; Diphenylether-4,4'-dicarbonsäure-(4-((ethenyloxy)methyl)-cyclohexyl)methanoldiester; Diphenylether-4,4'-dicarbonsäure-2-(ethenyloxy)ethanoldiester und 1,3,5,7-Tetra-[(4-((1-propenyloxy)methyl)cyclohexyl)methoxyethyl]-1,3,5,7-tetramethylcyclotetrasiloxan.

Dentalwerkstoffe sollten in bevorzugter Ausführungsform einen Brechungsindex aufweisen, der dem des natürlichen Materials nahe kommt. Von Vorteil ist weiterhin, wenn der Brechungsindex der Matrix dem des Füllstoffs in etwa entspricht, da andernfalls die Trübung des Werkstoffs ansteigt. Diese könnte auch zu Problemen bei einer möglichen Photohärtung des Dentalwerkstoffs führen. Um den Brechungsindex zu erhöhen, kann der Anteil aromatischer Gruppen in dem Bindemittel vergrößert werden. Darüber hinaus steigern auch Schwefelatome diesen Wert, wobei diese Atome beispielsweise durch Thioether-, Thioester- und/oder Thiolgruppen in Monomeren des Bindemittels vorhanden sein können.

Besonders bevorzugte Monomere der Formel (1) weisen ein Molekulargewicht im Bereich von 300 bis 3000 auf. Es wurde besonders überraschend festgestellt, daß Monomere die diesem Kriterium entsprechen, einen besonders geringen Schrumpf aufweisen, wobei der gehärtete Dentalwerkstoff gleichzeitig hervorragende mechanische Eigenschaften zeigt, wie hohe Druckbeständigkeit, hoher Elastizitätsmodul und hohe Biegefestigkeit, ohne jedoch spröde zu sein.

Einige Monomere der Formel (I) sind Polymere bzw. Oligomere. Aufgrund ihrer Herstellung weisen diese Verbindungen im allgemeinen eine Molekulargewichtsverteilung auf. Die zuvor angegebenen Werte des Molekulargewichts beziehen sich in diesen Fällen auf das Gewichtsmittel. Dieses kann beispielsweise durch Viskosimetrie bestimmt werden.

Die oben genannten Monomere sind Großteils kommerziell erhältlich, darüber hinaus können sie in an sich bekannter Weise synthetisch erhalten werden. Hierbei können die genannten Reste X und Y als Anhaltspunkte dienen. Hilfreiche Hinweise erfährt der Fachmann darüber hinaus beispielsweise aus Ullmann's Encyclopedia of Industrial Chemistry, 5. Aufl.

Die Herstellung von Vinylverbindungen aus Vinylhalogeniden und Alkoxiden wurde insbesondere von W. Reppe et al., *Justus Liebigs Ann. Chem.* **601** (1956) 81 - 110 beschrieben. Des weiteren sind Transvinylierungsreaktionen bekannt, bei denen eine Vinylgruppen eines Vinylethers oder Esters auf einen Alkohol bzw. eine Säure übertragen wird. Beispielhaft für viele seien folgende Literaturstellen genannt: N. D. Field et al. : "Vinyl Ethers," in *High Polymers,* vol. 24, Wiley, New York 1971, pp. 365 - 411; S. A. Miller : "Vinyl Ethers," in *Acetylene, Its Properties, Manufacture and Uses,* vol. 2, Ernest Benn Ltd., London 1966, pp. 198 - 231; D. H. Lorenz : *Encyclopedia of Polymer* Science *and Technology,* vol. 14, Interscience, New York 1971, pp. 504 - 510.

Des weiteren können Vinylgruppen aus Allylgruppen durch Isomerisierungsreaktionen erhalten werden. Die Isomerisierung kann sowohl durch Addition von Base als auch durch Einwirkung von Ru-Katalysatoren, wie (Ph₃P)₃RuCl₂, erfolgen.

Die oben genannten Siloxane können beispielsweise aus den entsprechenden Hydrogensiloxanen durch Hydrosilylierung mit (1-Propenoxy)vinyloxyalkanen erhalten werden. Hierbei reagiert hauptsächlich die Vinyloxygruppe mit der Si-H-Bindung des Hydrogensiloxans. Zur Katalyse dieser Reaktion werden im allgemeinen Pt, Rh, und/ oder Pd-Verbindungen eingesetzt. Bekannt sind unter anderem Karsted und Wilkinson-Katalysatoren. (1-Propenoxy)vinyloxyalkanen können beispielsweise durch Isomerisierung aus Allyloxyvinyloxyalkanen gewonnen werden.

Vorzugsweise beträgt die Viskosität des Bindemittels 1 mPa*s bis 6 Pa*s, vorzugsweise 1 mPa*s bis 1 Pa*s ohne daß hierdurch eine Beschränkung erfolgen soll. Der untere Grenzwert ergibt sich aus Flüchtigkeit des Bindemittels. Ist die Viskosität größer als 6 Pa*s, so läßt sich nur eine sehr begrenzte Menge an anorganischem Füllstoff einarbeiten. Ein hoher Füllstoffgehalt führt jedoch zu einem geringen Schrumpf, hervorragenden mechanischen Eigenschaften und zu einer guten Polierbarkeit des ausgehärteten Werkstoffs.

Zur Beeinflussung der Viskosität kann das Bindemittel des weiteren monofunktionale, kationisch mit Verbindungen der Formel (I) copolymerisierbare Monomere umfassen.

Hierzu gehören u.a. Vinylester, wie Vinylacetat, Vinylether, wie z.B. Propylvinylether, Butylvinylether, 2-Methylpropylvinylether, Pentylvinylether, Hexylvinylether, ((1-Propenoxy)ethyl)trimethylsilan, ((1-Propenoxy)ethyl)triethylsilan, und Vinylaromaten, wie z.B. Styrol, substituierte Styrole mit einem Alkylsubstituenten in der Seitenkette, wie z.B. α-Methylstyrol und α-Ethylstyrol, substituierte Styrole mit einem Alkylsubstituenten an dem Ring, wie beispielsweise Vinyltoluol und p-Methylstyrol, halogenierte Styrole, wie beispielsweise Monochlorstyrole, Dichlorstyrole, Tribromstyrole und Tetrabromstyrole.

Zur Härtung des Dentalwerkstoffs wird ein Polymerisationsinitiator zugesetzt. Vorzugsweise erfolgt die Polymerisation kationisch. Dementsprechend enthalten besonders bevorzugte Initiatoren Lewis- oder Brönstett-Säuren bzw. Verbindungen, die solche Säuren freisetzen, wie beispielsweise BF₃ oder dessen etherische Addukte (BF₃·THF, BF₃·Et₂O, usw.), AlCl₃, FeCl₃, HPF₆, HAsF₆, HSbF₆, HBF₄, denen unter Umständen ein halogenierter Kohlenstoff, wie beispielsweise Triphenylchlormethan zugegeben wird, oder Substanzen, die nach Bestrahlen durch UV oder sichtbares Licht oder durch Wärme und/oder Druck die Polymerisation auslösen, wie z.B. (eta-6-Cumol)(eta-5-cyclopentadienyl)eisen-hexafluorophosphat, (eta-6-Cumol)(eta-5-cyclopentadienyl)eisen-tetrafluoroborat, substituierte Diaryliodoniumsalze und Triarylsulfoniumsalze.

Diesen Initiatoren werden üblicherweise Beschleuniger bzw. Sensibilisatoren, wie beispielsweise Peroxyverbindungen, insbesondere vom Perestertyp, Benzoinderivate, Benzilverbindungen, oder Acylphosphinoxide beigefügt. Das Verhältnis von Initiator zu Beschleuniger kann in weiten Grenzen von 1 : 0,001 bis 1:10 variiert werden, vorzugsweise wird jedoch ein Verhältnis von 1:0,1 bis 3:6 verwendet.

Besonders bevorzugte Polymerisationsinitiatoren enthalten Iodoniumsalze als Initiatoren sowie Benzoinderivate, wie Benzoin, α-Methylbenzoinmethylether, α-Dicarbonylverbindungen, wie 2,3-Butandion, Campherchinon, Benzil und dessen Derivate, wie ω,ω-Dimethoxy-ω-phenylacetophenon, α-Hydroxyalkylphenonderivate, wie 1-Benzolycyclohexan-1-ol, sowie Acylphosphinoxidverbindungen, wie Benzoyldiphenylphosphinoxid, Trimethylbenzoyldiphenylphosphinoxid (weitere sind in EP 0 073 413 beschrieben) als Sensibilisatoren. Teilweise müssen Amine als zusätzliche Coinitiatoren zugesetzt werden.

Besonders geeignete Diaryiodoniumverbindungen sind beispielsweise Diphenyliodoniumtetrafluoroborat, Diphenyiodoniumhexafluorophosphat, Bis-(4-methylphenyl) iodoniumhexafluorophosphat, Dinaphthyliodoniumhexafluoroantimonat, und Phenyl-4-methylphenyliodoniumhexafluoroantimonat.

Die Iodoniumsalze enthaltenden Polymerisationsinitiatoren, die im allgemeinen Sensibilisatoren enthalten, lassen sich häufig durch sichtbares Licht im Wellenlängenbereich von 380 bis 500 Nanometer initiieren. Überraschend wurde festgestellt, daß diese Initiatoren Verbindungen der Formel (1) besonders effektiv und schnell härten. Hierbei ist es nicht notwendig den Werkstoff während der gesamten Härtungszeit zu bestrahlen, da nach einer ausreichenden Initiierung der Dentalwerkstoff vollständig durchhärtet. Diese Eigenschaft ist besonders bei dem Einbringen von Füllungen nützlich.

Die Verwendung anorganischer Füllstoffe in Dentalwerkstoffen ist an sich bekannt. Die Füllstoffe dienen insbesondere zur Verbesserung der mechanischen Eigenschaften. Als Füllstoff werden unter anderem Quarze, gemahlene Gläser, Aerosile, sphärische SiO₂-Partikel, die ggf. mit Titandioxid beschichtet sind, Zeolithe, Ormocere, Ormosile, schwer lösliche Fluoride, wie CaF₂, YF₃, Kieselgele sowie pyrogene Kieselsäuren oder deren Granulate weithin angewendet. Des weiteren können die erfindungsgemäßen Dentalwerkstoffe auch organische Füllstoffe, insbesondere Fasern aufweisen. Diese Füllstoffe sind im allgemeinen kommerziell erhältlich.

Insbesondere schwere Atome, wie Y und Zr, erhöhen die Röntgenopazität. Daher werden Füllstoffe bevorzugt, die diese Atome aufweisen.

Zum besseren Einbau in die Polymermatrix können diese Füllstoffe mit Haftverbesserungsmitteln behandelt werden. Hierzu sind unter anderem Silane, wie ((1-Propenoxy)ethyl)-trimethoxysilan, ((1-Propenoxy)ethyl)triethoxysilan oder (1-Propenoxy)ethyltrichlorsilan, geeignet.

Ohne daß hierdurch eine Beschränkung erfolgen soll weisen diese Füllstoffe im allgemeinen eine Korngröße im Bereich von 0,02 µm bis 100 µm, bevorzugt von 0,05 bis 10 µm und ganz besonders bevorzugt von 0,1 bis 5 um auf, wobei die Form der Füllstoffe keiner besonderen Beschränkung unterliegt. Sie können dementsprechend beispielsweise kugelförmig, splitterförmig, plättchenförmig und/oder faserförmig sein.

Diese Füllstoffe können einzeln oder als Mischungen eingesetzt werden, wobei der Einsatz von Mischungen unter Umständen Verbesserungen hinsichtlich der Ästhetik und eine weitere Verbesserung der mechanischen Eigenschaften ermöglicht.

Der Füllstoffgehalt erfindungsgemäßer Dentalwerkstoffe liegt im Bereich von 1 bis 95 Gew.-%, bevorzugt im Bereich von 50 bis 90 Gew.-% und ganz besonders bevorzugt im Bereich von 65 bis 90 Gew.-%, bezogen auf das Gesamtgewicht. Ein hoher Füllstoffgehalt führt zu einem geringen Schrumpf, hervorragenden mechanischen Eigenschaften und zu einer guten Polierbarkeit des ausgehärteten Werkstoffs. Andererseits muß in dem Dentalwerkstoff genügend Bindemittel zur Härtung vorhanden sein. Je gleichmäßiger das Bindemittel in den Füllstoff eingearbeitet werden kann, desto höher kann der Füllstoffgehalt gewählt werden.

Darüber hinaus können die Dentalwerkstoffe der vorliegenden Erfindung Hilfsstoffe aufweisen. Hierzu gehören unter anderem Stabilisatoren, Pigmente oder Verdünnungsmittel.

Ein besonders bevorzugter Dentalwerkstoff der vorliegenden Erfindung besteht beispielsweise aus

| | |
|---|---|
| 4,98 - 95 Gew.-% | Bindemittel, |
| 0,02 - 10 Gew.-% | Polymerisationsinitiator, |
| 1 - 95 Gew.-% | Füllstoff und |
| 0 - 20 Gew.-% | üblichen Additive, jeweils bezogen auf das Gesamtgewicht des Dentalwerkstoffs. |

Der erfindungsgemäße Dentalwerkstoff kann in an sich bekannter Weise hergestellt werden. Hierbei werden die Komponenten gemischt, wobei der Füllstoff vorzugsweise in Portionen zugegeben wird.

Der ausgehärtete Dentalwerkstoff zeigt hervorragende Eigenschaften hinsichtlich der Biegefestigkeit gemäß DIN 53 452, des Elastizitätsmoduls gemäß DIN 53 457, der Druckfestigkeit, der Haltbarkeit und der Abriebfestigkeit, so ist die Biegefestigkeit bevorzugter Ausführungsformen im allgemeinen ≥ 100 N/mm², und der E-Modul vorzugsweise ≥ 8000 N/mm².

Darüber hinaus zeigen die Dentalwerkstoffe der vorliegenden Erfindung eine ausgezeichnete Polierbarkeit, eine geringe Wasseraufnahme und hervorragende ästhetische Eigenschaften, insbesondere hinsichtlich der Transparenz und des Brechungsindexes.

Die nachfolgenden Beispiele und Vergleichsbeispiele dienen zur genaueren Erläuterung der Erfindung, ohne daß diese hierauf beschränkt werden soll. Die nachfolgenden Prozentangaben beziehen sich auf das Gesamtgewicht, es sei denn anderes ist vermerkt.

### Beispiel 1

Herstellung von 1,3,5,7-Tetra-[(4-((1-propenyloxy)methyl)-cyclohexyl)methoxyethyl] -1,3,5,7-tetramethylcyclotetrasiloxan (DEB 461)

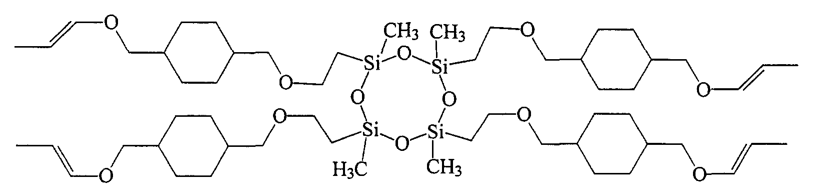

In einen mit Thermometer, Rührer und Rückflußkühler ausgestatteten 500 ml Dreihalskolben werden 0,5 Mol (84,5 g) 4-(Hydroxymethyl)-1-(vinyloxymethyl)-cyclohexan, 0,65 Mol (78,64 g) Allylbromid, 0,65 Mol (26 g) NaOH, 0,31 Mol (10 g) Tetra-n-butylammoniumbromid und 200 ml Toluol gegeben. Nach 15 Minuten Rühren unter Stickstoffatmosphäre wird die Mischung auf 90°C für 16 Stunden erhitzt. Danach wird auf Raumtemperatur gekühlt und die Mischung in 300 ml Wasser gegeben. Die organische Phase wird abgetrennt und zweimal mit Wasser gewaschen. Anschließend wird das Lösungsmittel unter vermindertem Druck durch Destillation entfernt. Der Rückstand wird mittels Chromatographie gereinigt, wobei Silika-Gel und eine 1:9 Mischung aus Ethylacetat und Hexan verwendet wird.

Das so erhaltene Allylvinylalkan wird anschließend durch Isomerisierung in das 1-Propenyl-Derivat überführt. Hierbei wird das zuvor erhaltene Produkt in einen Rundkolben gegeben, der mit Rückflußkühler und Rührer ausgestattet ist. Nach Zugabe von 0,64 µmol (0,06g) (Ph₃P)₃RuCl₂ wird die Mischung 8 Stunden auf 130 °C erwärmt. Das erhaltene Produkt wird anschließend bei reduziertem Druck destilliert (93°C bei etwa 26 Pa). Es wurden insgesamt 0,435 Mol (91,8 g) 4-(1-Propenyloxymethyl)-1-(vinyloxymethyl)-cyclohexan erhalten (ca. 87% Ausbeute).

Das 4-(1-Propenyloxymethyl)-1-(vinyloxymethyl)-cyclohexan wird anschließend mit Si-H-funktionellem 1,3,5,7-Tetramethylcyclotetrasiloxan umgesetzt.

Hierzu werden 0,435 Mol (91,8 g) des Divinylethers, 0,1 Mol (24 g) des Cyclosiloxan und 100 ml trockenes Toluol in einen Rundkolben gegeben. Anschließend fügt man 5 µl Karsteds Katalysator bei (3% Pt-Komplex in Xylol gemäß US-PS 3 814 730). Die Mischung wird zunächst bei 50°C unter Stickstoffatmosphäre gerührt. Anschließend erhitzt sich die Mischung auf 100°C durch freigesetzte Reaktionswärme. Nach 16 Stunden ist die Umsetzung beendet. Danach werden die Edukte und das Lösungsmittel mittels Destillation abgetrennt, wobei Reste im Hochvakuum entfernt werden.

Das erhaltene Produkt (DEB 461) wurde mittels NMR-Spektroskopie und Elementaranalyse charakterisiert und ohne weitere Aufreinigung verwendet. Die Ausbeute beträgt etwa 98% (105,8 g).

### Beispiel 2

11,88 g DEB 461, wie in Beispiel 1 hergestellt, werden mit 0,0839 g (0,7%) Iodoniumsalz (4-(n-Octyloxy)phenyl)phenyl-iodoniumhexafluoroantimonat)), 0,0248 g Sensibilisator (Acylphosphinoxid, ® Igacure 819) und 0,0024 g (0,002%) Stabilisator (® BHT Ralox) gemischt.

Die so hergestellte Mischung wird für 20 Sekunden mit Blaulicht bestahlt, wodurch diese vollständig härtet. Die so gehärtete Kunststoffmatrix wird auf seine mechanischen Eigenschaften und die Volumenabnahme untersucht, die durch die Polymerisation eintritt (Schrumpf). Die erhaltenen Ergebnisse sind in Tabelle 1 dargestellt.

### Beispiel 3

Um aus der in Beispiel 2 erhaltenen Mischung einen Dentalwerkstoff herzustellen, werden in 11,99 g der härtbaren Monomermischung zunächst insgesamt 0,645 g Aerosil in zwei Portionen zugegeben. Anschließend wird mit Vinyloxyethyl-N-(3-(triethoxysilyl)propyl)carbamat (VES) silanisiertes Glas eingeknetet. Die Zugabe von insgesamt 24,81 g erfolgt in neun Schritten (1: 9,99 g, 2: 5,04 g, 3: 2,49 g, 4: 1,99 g, 5: 2,07 g, 6: 1,63 g, 7: 0,83 g, 8: 0,54 g und 9: 0,23 g), wobei ein Kleinstkneter bei einer Knetzeit von jeweils 10 min und einem Druck von 180 bis 210 mbar verwendet wird.

### Vergleichsbeispiel 1

Herstellung von 1,3,5,7-Tetra-[(4-((oxymethacroyl)methyl)-cyclohexyl)methoxyethyl]-1,3,5,7-tetramethylcyclotetrasiloxan (DEB 502)

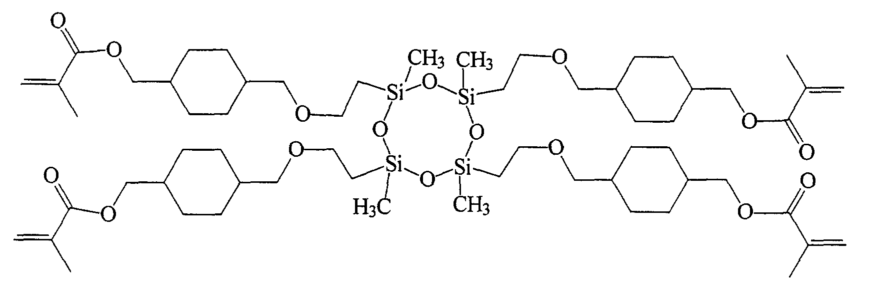

Die Herstellung von DEB 502 wird auf ähnliche Weise wie die Darstellung von DEB 461 durchgeführt. Allerdings wird 4-(Hydroxymethyl)-1-(vinyloxymethyl)-cyclohexan zunächst mit 1,3,5,7-Tetramethylcyclotetrasiloxan umgesetzt. Anschließend wird das Produkt mit Methacrylsäureanhydrid zu DEB 502 umgesetzt.

### Vergleichsbeispiel 2

Vergleichsbeispiel 2 wird auf ähnliche Weise wie Beispiel 2 durchgeführt, außer daß man DEB 502 anstatt DEB 461 verwendet. Dementsprechend wird ein radikalisches Initiatorsystem bestehend aus Campherchinon und Speedcure EDB eingesetzt.

Das Bindemittel wird des weiteren 60 Sekunden gehärtet, um einen vollständigen Umsatz sicherzustellen. Diese Kunststoffmatrix wird ebenfalls auf ihre mechanischen Eigenschaften und den Schrumpf untersucht. Die erhaltenen Werte sind in Tabelle 1 aufgeführt.

### Beispiel 4

Das Beispiel 4 wird auf gleiche Weise wie Beispiel 2 durchgeführt, außer daß 11,88 g Diphenylether-4,4'-dicarbonsäure-(4-(vinyloxymethyl)cyclohexyl)methanoldiester (CHMVEE) anstatt DEB 461 eingesetzt wird, welches in Anlehnung an Beispiel 1 erhalten wurde

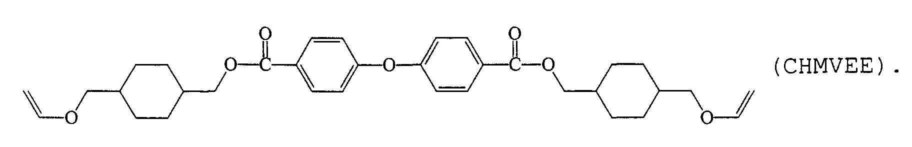

Nach der Härtung wird die Kunststoffmatrix auf ihre mechanischen Eigenschaften und auf Schrumpf untersucht. Die erhaltenen Ergebnisse sind in Tabelle 1 dargestellt.

**Tabelle 1:**

| Mechanische Eigenschaften der Kunststoffmatrizes | | | |
|---|---|---|---|
| | Schrumpf (ΔV) [%] | Biegefestigkeit (DIN 53452) [N/mm²] | E-Modul (DIN 53457) [N/mm²] |
| Beispiel 2 | 2,7761 | 50 | 1200 |
| Vergleichsbsp. 2 | 5,2198 | 40 | 800 |
| Beispiel 4 | 3,8315 | 80 | 2000 |

## Patentansprüche

1. Dentalwerkstoff auf Basis von kationisch polymerisierbaren, Monomeren als Bindemittel, eines Polymerisationsinitiators und bezogen auf den Dentalwerkstoff 1-95 Gew.-% mindestens eines anorganischen Füllstoffs,
**dadurch gekennzeichnet,**
**daß** das Bindemittel Monomere der Formel (I) enthält 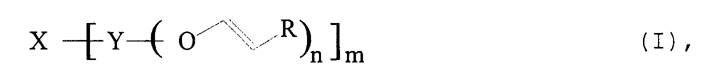 worin R Wasserstoff, eine Methyl- oder Ethylgruppe, X und Y jeweils unabhängig einen unsubstituierten oder substituierten aliphatischen, cycloaliphatischen oder aromatischen Rest mit 1 bis 100 Kohlenstoffatomen, wobei eine oder mehrere CH₂-Gruppen durch O, C=O, -CO₂-, -SiR¹₂- und/ oder -SiR¹₂O-, worin R¹ unabhängig ein Alkyl- oder Alkoxy- oder Arylrest mit 1 bis 10 C-Atomen bedeutet, ersetzt sein können, n eine ganze Zahlen von 1 bis 3 und m eine ganze Zahl von 2 bis 5 darstellen.

2. Dentalwerkstoff gemäß Anspruch 1, **dadurch**
**gekennzeichnet, daß** der Rest X einen oder mehreren der folgenden Gruppen aufweist 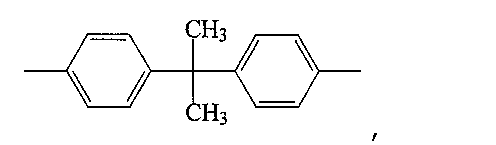 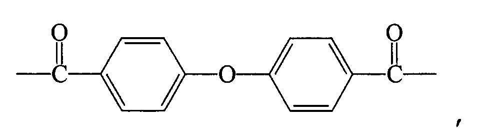 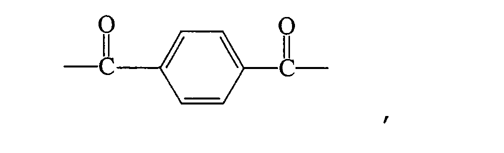 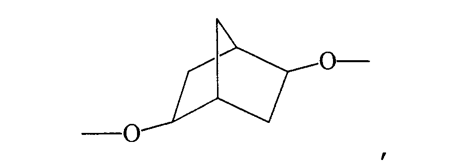 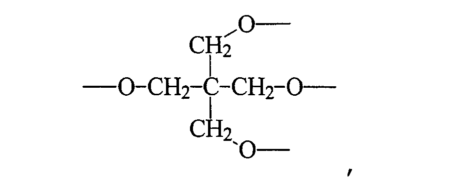 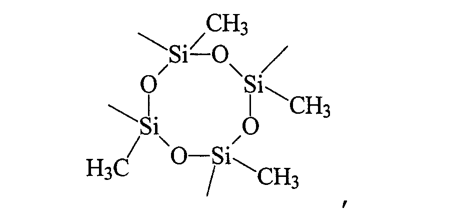 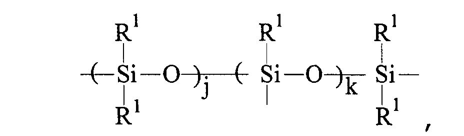 worin R¹ unabhängig ein Alkyl- oder Alkoxyrest mit 1 bis 6 C-Atomen, j und k unabhängig ganze Zahlen im Bereich von 1 bis 10 darstellen.

3. Dentalwerkstoff gemäß Anspruch 1 oder 2, **dadurch gekennzeichnet, daß** der Rest Y einen oder mehreren der folgenden Gruppen aufweist 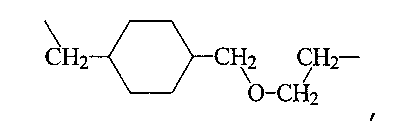 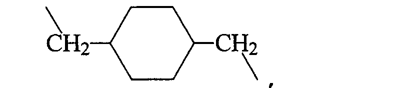 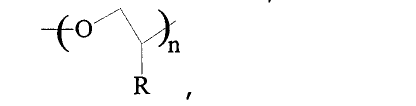 worin R Wasserstoff oder Methyl und n eine ganze Zahl im Bereich von 1 bis 10 darstellt.

4. Dentalwerkstoff gemäß einem oder mehreren der vorhergehenden Ansprüche, **dadurch gekennzeichnet, daß** die Monomere der Formel (I) ein Molekulargewicht im Bereich von 300 bis 3000 aufweisen.

5. Dentalwerkstoff gemäß einem oder mehreren der vorhergehenden Ansprüche, **dadurch gekennzeichnet, daß** das Bindemittel eine Viskosität im Bereich von 1 mPa*s bis 1000 mPa*s aufweist.

6. Dentalwerkstoff gemäß einem oder mehreren der vorhergehenden Ansprüche, **dadurch gekennzeichnet, daß** das Bindemittel zusätzlich monofunktionale Vinylether aufweist.

7. Dentalwerkstoff gemäß einem oder mehreren der vorhergehenden Ansprüche, **dadurch gekennzeichnet, daß** der Polymerisationsinitiator ein Iodoniumsalz und einen Sensibilisator enthält.

8. Dentalwerkstoff gemäß einem oder mehreren der vorhergehenden Ansprüche, **dadurch gekennzeichnet, daß** der Polymerisationsinitiator durch Bestrahlen mit sichtbarem Licht initiiert werden kann.

9. Dentalwerkstoff gemäß einem oder mehreren der vorhergehenden Ansprüche, **dadurch gekennzeichnet, daß** der Füllstoff Quarz, gemahlenes Glas, Kieselgel, Kieselsäure, einen Zeolith oder ein Ormocer sowie Mischungen dieser Stoffe aufweist.

10. Dentalwerkstoff gemäß Anspruch 9, **dadurch gekennzeichnet, daß** der Füllstoff mit einem Haftvermittler behandelt ist.

11. Dentalwerkstoff gemäß einem oder mehreren der vorhergehenden Ansprüche, **dadurch gekennzeichnet, daß** der Füllstoffgehalt im Bereich von 50 bis 90 Gew.-% und ganz besonders bevorzugt im Bereich von 65 bis 90 Gew.-% liegt, bezogen auf das Gesamtgewicht.

12. Dentalwerkstoff gemäß einem oder mehreren der vorhergehenden Ansprüche, **dadurch gekennzeichnet**,
**dadurch gekennzeichnet, daß** die Biegefestigkeit gemäß DIN 53 452 ≥ 30 N/mm² und/oder der Elastizitätsmodul gemäß DIN 53 457 ≥ 500 N/mm² ist.

13. Dentalwerkstoff gemäß einem oder mehreren der vorhergehenden Ansprüche, **dadurch gekennzeichnet, daß** der Werkstoff aus
4,98 - 95 Gew.-% Bindemittel,
0,02 - 10 Gew.-% Polymerisationsinitiator,
1 - 95 Gew.-% Füllstoff und
0 - 20 Gew.-% üblichen Additive, jeweils bezogen auf das Gesamtgewicht des Dentalwerkstoffs, besteht.

14. Verfahren zur Herstellung eines Dentalwerkstoffs gemäß den Ansprüchen 1 bis 13, **dadurch gekennzeichnet, daß** man die Komponenten mischt.
